# FASCICULE DE BREVET EUROPEEN

(11) **EP 3 852 703 B1**
(45) Date de publication et mention de la délivrance du brevet: **20.09.2023**
(21) Numéro de dépôt: 19769809.5
(22) Date de dépôt: 20.09.2019
(51) Int. Cl.: A61F 9/008

(54) **PROCEDE ET DISPOSITIF DE DETERMINATION D'UNE VALEUR MINIMALE D'ENERGIE LASER NECESSAIRE A LA FORMATION D'UNE BULLE DE GAZ**
VERFAHREN UND VORRICHTUNG ZUR BESTIMMUNG EINES MINDESTWERTS DER ZUR BILDUNG EINER GASBLASE NOTWENDIGEN LASERENERGIE
METHOD AND DEVICE FOR DETERMINING A MINIMUM VALUE OF LASER ENERGY NECESSARY FOR THE FORMATION OF A GAS BUBBLE

(30) Priorité: 20.09.2018 FR 1858534
(43) Date de publication de la demande: 28.07.2021
(73) Titulaire: Keranova, 42000 Saint Etienne (FR)
(72) Inventeur: BERNARD, Aurélien, 42000 SAINT ETIENNE (FR); BAUBEAU, Emmanuel, 42000 SAINT ETIENNE (FR)
(74) Mandataire: Be IP
(86) Numéro de dépôt international: PCT/EP2019/075280
(87) Numéro de publication internationale: WO 2020/058459

(56) Documents cités:
- WO-A1-2015/069197
- RU-A- 2009 137 641
- RU-C2- 2 424 785
- US-A1- 2012 259 321

## Description

### DOMAINE TECHNIQUE

La présente invention concerne le domaine technique des opérations chirurgicales réalisées au laser femtoseconde, et plus particulièrement celui de la chirurgie ophtalmologique, notamment pour des applications de découpes de cornées ou de cristallins.

L'invention concerne un dispositif de découpe d'un tissu humain ou animal, telle qu'une cornée, ou un cristallin, au moyen d'un laser femtoseconde.

Par laser femtoseconde, on entend une source lumineuse, apte à émettre un faisceau L.A.S.E.R. sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

Les lasers femtoseconde sont couramment utilisés en chirurgie pour la découpe de la cornée ou du cristallin. Ils délivrent des impulsions ultra-brèves et de forte puissance.

Lors d'une chirurgie du cristallin, le laser femtoseconde peut être utilisé pour réaliser une découpe du tissu cornéen, en focalisant un faisceau L.A.S.E.R. dans le cristallin. Plus précisément, à chaque impulsion, le laser femtoseconde génère un faisceau. Ce faisceau est focalisé (en un point dit « *de focalisation »* situé) dans le cristallin. Une bulle de gaz se forme au point de focalisation, engendrant une disruption très localisée des tissus environnant.

Une partie de l'énergie du faisceau est consommée lors de la génération de la bulle de gaz. Le reste de l'énergie du faisceau se propage jusqu'à la rétine, ce qui provoque un échauffement local de la rétine, qui pourrait générer une lésion.

Pour limiter l'échauffement local de la rétine, il est donc préférable que l'énergie du faisceau n'excède pas l'énergie minimale nécessaire à la formation d'une bulle de gaz.

Il est très difficile de déterminer cette énergie minimale nécessaire à la formation d'une bulle de gaz en utilisant uniquement des données préopératoires. En effet, la quantité d'énergie nécessaire à la formation d'une bulle de gaz dans le cristallin dépend de nombreux facteurs tels que :
- les inhomogénéités du cristallin, en particulier du cristallin cataracté où la transparence est réduite de manière hétérogène (le cristallin peut être plus ou moins opaque à certains endroits),
- la diffusion du faisceau L.A.S.E.R. lors de sa propagation,
- les inhomogénéités de qualité du faisceau L.A.S.E.R. dues aux imperfections des éléments optiques.

Les méthodes actuelles visant à réaliser au moyen d'un L.A.S.E.R. femtoseconde une découpe d'un tissu oculaire comme par exemple un cristallin humain cataracté mettent en oeuvre des moyens empiriques de détermination de l'énergie nécessaire à obtenir un résultat optimal. Il est donc courant que l'énergie employée ne soit pas adaptée en tout point du tissu à découper et que l'énergie soit trop élevée ou pas assez élevée, ce qui résulte respectivement en la formation de bulles de gaz trop volumineuses ou à l'absence d'efficacité. La formation de bulles de gaz trop volumineuses peut avoir pour conséquence la rupture du sac capsulaire, complication chirurgicale importante, ou le manque d'efficacité de la suite de la découpe en raison de l'atténuation de l'efficacité du laser due au rideau de bulles qui peut se former. Le manque d'efficacité si l'énergie n'est pas assez élevée se traduit pour le chirurgien par la nécessité de recourir plus longtemps à l'utilisation d'ultrasons pour terminer la fragmentation du cristallin cataracté.

US 2012/259321 divulgue un dispositif de détermination d'une valeur minimale d'énergie L.A.S.E.R. nécessaire pour former, en utilisant un appareil de découpe incluant une source L.A.S.E.R. femtoseconde, une bulle de gaz dans au moins une zone élémentaire s'étendant dans un plan de découpe d'un tissu oculaire, le dispositif de détermination incluant un système d'imagerie pour l'acquisition d'images.

Il est donc nécessaire de mettre au point une méthode non empirique qui détermine en chaque point du tissu à découper, le niveau d'énergie optimale permettant d'obtenir un traitement efficace avec un niveau de sécurité optimal.

Un but de la présente invention est donc de proposer un procédé et un dispositif associé de détermination d'une valeur minimale d'énergie L.A.S.E.R. nécessaire pour former une bulle de gaz dans une (ou plusieurs) zone(s) élémentaire(s) d'un tissu oculaire.

### EXPOSE DE L'INVENTION

A cet effet l'invention propose un procédé de détermination d'une valeur minimale d'énergie L.A.S.E.R. nécessaire pour former, en utilisant un appareil de découpe incluant une source L.A.S.E.R. femtoseconde, une bulle de gaz dans au moins une zone élémentaire s'étendant dans un plan de découpe d'un tissu oculaire, ***remarquable en ce que*** le procédé comprend les étapes suivantes :
a) Réception par une unité de traitement de données, d'une image de référence du tissu oculaire, ladite image de référence ayant été acquise par un système d'imagerie antérieurement à l'émission d'une pluralité de faisceaux L.A.S.E.R. aptes à focaliser en au moins un point d'échantillonnage de chaque zone élémentaire, chaque faisceau L.A.S.E.R. ayant une énergie respective différente des autres faisceaux L.A.S.E.R. de la pluralité de faisceaux L.A.S.E.R.,
b) Réception par l'unité de traitement de données, d'au moins une image courante du tissu oculaire, chaque image courante ayant été acquise par le système d'imagerie postérieurement à l'émission d'au moins un faisceau L.A.S.E.R. de la pluralité de faisceaux L.A.S.E.R., puis pour chaque zone élémentaire, mise en oeuvre des étapes suivantes par l'unité de traitement de données :
c) Détection en comparant l'image de référence à chaque image courante, d'au moins une bulle de gaz formée dans la zone élémentaire, chaque bulle de gaz étant associée à un faisceau L.A.S.E.R. respectif,
d) Détermination en fonction de l'au moins une bulle de gaz détectée, d'une valeur minimale d'énergie nécessaire à la formation d'une bulle de gaz dans la zone élémentaire.

Des aspects préférés mais non limitatifs du procédé selon l'invention sont les suivants :
- les images de référence et courante(s) peuvent être des images OCT, l'étape de détection comprenant, pour chaque image courante, les sous-étapes consistant à :
   - calculer en chaque point d'échantillonnage, une variation d'intensité de lumière rétrodiffusée reçue par le système d'imagerie entre les pixels de l'image courante et les pixels de l'image de référence,
   - comparer cette variation d'intensité calculée à une valeur seuil pour identifier la formation d'une bulle de gaz si la variation d'intensité calculée est supérieure à la valeur seuil ;
- pour chaque zone élémentaire, chaque faisceau L.A.S.E.R. peut être apte à focaliser en un unique point d'échantillonnage de la zone élémentaire, l'étape b) de réception consistant à recevoir une pluralité d'images courantes du tissu oculaire, chaque image courante ayant été acquise par le système d'imagerie postérieurement à l'émission d'un faisceau L.A.S.E.R. respectif dans chaque zone élémentaire ;
- l'étape de détermination peut inclure les sous-étapes suivantes pour chaque zone élémentaire :
   - si une unique bulle de gaz est détectée :
      ▪ assigner à la valeur minimale, un multiple k de l'énergie du faisceau L.A.S.E.R. ayant induit la formation de la bulle de gaz détectée, k étant un nombre décimal compris entre 1 et 2,
   - si plusieurs bulles de gaz sont détectées :
      ▪ sélectionner parmi les images courantes dans lesquelles une bulle de gaz est détectée, l'image courante associée au faisceau L.A.S.E.R. d'énergie la plus faible, et
      ▪ assigner à la valeur minimale, un multiple k de l'énergie du faisceau L.A.S.E.R. associé à l'image courante sélectionnée, k étant un nombre décimal compris entre 1 et 2 ;
- pour chaque zone élémentaire, chaque faisceau L.A.S.E.R. peut être apte à focaliser en un point d'échantillonnage respectif de la zone élémentaire, l'étape b) de réception consistant à recevoir une image courante du tissu oculaire, ladite image courante ayant été acquise par le système d'imagerie postérieurement à l'émission de la pluralité de faisceaux L.A.S.E.R. en un point d'échantillonnage respectif de chaque zone élémentaire ;
- l'étape de détermination peut inclure les sous-étapes suivantes pour chaque zone élémentaire :
   - si une unique bulle de gaz est détectée :
      ▪ assigner à la valeur minimale, un multiple k de l'énergie du faisceau L.A.S.E.R. associé au point d'échantillonnage au niveau duquel la bulle de gaz est détectée, k étant un nombre décimal compris entre 1 et 2,
   - si plusieurs bulles de gaz sont détectées :
      ▪ sélectionner parmi les points d'échantillonnage au niveau de chacun desquels une bulle de gaz est détectée, le point d'échantillonnage associé au faisceau L.A.S.E.R. d'énergie la plus faible, et
      ▪ assigner à la valeur minimale, un multiple k de l'énergie du faisceau L.A.S.E.R. associé audit point d'échantillonnage sélectionné, k étant un nombre décimal compris entre 1 et 2 ;
- le procédé peut comprendre en outre l'étape suivante :
   e) enregistrement par l'unité de traitement de données, de la valeur minimale déterminée pour chaque zone élémentaire dans une mémoire ;
- le tissu oculaire peut comprendre un ensemble de zones élémentaires s'étendant dans une pluralité de plans de découpe contenant chacun au moins une zone élémentaire, le procédé comprenant la réitération des étapes a) à e) pour chaque plan de découpe afin de déterminer une carte tridimensionnelle des valeurs minimales d'énergie nécessaire à la formation de bulles de gaz dans l'ensemble des zones élémentaires du tissu oculaire.

L'invention concerne également un dispositif de détermination d'une valeur minimale d'énergie L.A.S.E.R. nécessaire pour former, en utilisant un appareil de découpe incluant une source L.A.S.E.R. femtoseconde, une bulle de gaz dans au moins une zone élémentaire s'étendant dans un plan de découpe d'un tissu oculaire, le dispositif de détermination incluant un système d'imagerie pour l'acquisition d'images, ***remarquable en ce que*** le dispositif de détermination comprend en outre une unité de traitement de données incluant des moyens pour :
a) Recevoir une image de référence du tissu oculaire, ladite image de référence ayant été acquise par le système d'imagerie antérieurement à l'émission d'une pluralité de faisceaux L.A.S.E.R. aptes à focaliser en au moins un point d'échantillonnage de chaque zone élémentaire, chaque faisceau L.A.S.E.R. ayant une énergie respective différente des autres faisceaux L.A.S.E.R. de la pluralité,
b) Recevoir au moins une image courante du tissu oculaire, chaque image courante ayant été acquise par le système d'imagerie postérieurement à l'émission d'au moins un faisceau L.A.S.E.R. de la pluralité de faisceaux L.A.S.E.R.,
puis pour chaque zone élémentaire :
c) Détecter en comparant l'image de référence à chaque image courante, au moins une bulle de gaz formée dans la zone élémentaire, chaque bulle de gaz étant associée à un faisceau L.A.S.E.R. respectif,
d) Déterminer en fonction de l'au moins une bulle de gaz, une valeur minimale d'énergie nécessaire à la formation d'une bulle de gaz dans la zone élémentaire.

Des aspects préférés mais non limitatifs du dispositif selon l'invention sont les suivants :
- les images de référence et courante(s) peuvent être des images OCT, l'unité de traitement de données incluant des moyens pour détecter au moins une bulle de gaz en, pour chaque image courante:
   - calculant en chaque point d'échantillonnage, une variation d'intensité de lumière rétrodiffusée reçue par le système d'imagerie entre les pixels de l'image courante et les pixels de l'image de référence,
   - comparant cette variation d'intensité calculée à une valeur seuil pour identifier la formation d'une bulle de gaz si la variation d'intensité calculée est supérieure à la valeur seuil ;
- pour chaque zone élémentaire, chaque faisceau L.A.S.E.R. peut être apte à focaliser en un unique point d'échantillonnage de la zone élémentaire, l'unité de traitement de données incluant des moyens pour recevoir une pluralité d'images courantes du tissu oculaire, chaque image courante ayant été acquise par le système d'imagerie postérieurement à l'émission d'un faisceau L.A.S.E.R. respectif dans chaque zone élémentaire ;
- l'unité de traitement de données peut inclure des moyens pour déterminer une valeur minimale en, pour chaque zone élémentaire :
   - si une unique bulle de gaz est détectée :
      ▪ assignant à la valeur minimale, un multiple k de l'énergie du faisceau L.A.S.E.R. ayant induit la formation de la bulle de gaz détectée, k étant un nombre décimal compris entre 1 et 2,
   - si plusieurs bulles de gaz sont détectées :
      ▪ sélectionnant parmi les images courantes dans lesquelles une bulle de gaz est détectée, l'image courante associée au faisceau L.A.S.E.R. d'énergie la plus faible, et
      ▪ assignant à la valeur minimale, un multiple k de l'énergie du faisceau L.A.S.E.R. associé à l'image courante sélectionnée, k étant un nombre décimal compris entre 1 et 2 ;
- pour chaque zone élémentaire, chaque faisceau L.A.S.E.R. peut être apte à focaliser en un point d'échantillonnage respectif de la zone élémentaire, l'unité de traitement incluant des moyens pour recevoir une image courante du tissu oculaire, ladite image courante ayant été acquise par le système d'imagerie postérieurement à l'émission de la pluralité de faisceaux L.A.S.E.R. en un point d'échantillonnage respectif de chaque zone élémentaire ;
- l'unité de traitement de données peut inclure des moyens pour déterminer une valeur minimale en, pour chaque zone élémentaire :
   - si une unique bulle de gaz est détectée :
      ▪ assignant à la valeur minimale, un multiple k de l'énergie du faisceau L.A.S.E.R. associé au point d'échantillonnage au niveau duquel la bulle de gaz est détectée, k étant un nombre décimal compris entre 1 et 2,
   - si plusieurs bulles de gaz sont détectées :
      ▪ sélectionnant parmi les points d'échantillonnage au niveau de chacun desquels une bulle de gaz est détectée, le point d'échantillonnage associé au faisceau L.A.S.E.R. d'énergie la plus faible, et
      ▪ assignant à la valeur minimale, un multiple k de l'énergie du faisceau L.A.S.E.R. associé audit point d'échantillonnage sélectionné, k étant un nombre décimal compris entre 1 et 2 ;
- l'unité de traitement peut inclure en outre des moyens pour :
   e) enregistrer la valeur minimale déterminée pour chaque zone élémentaire dans une mémoire ;
- le tissu oculaire peut comprendre un ensemble de zones élémentaires s'étendant dans une pluralité de plans de découpe contenant chacun au moins une zone élémentaire, l'unité de traitement inclut en outre des moyens réitérer les étapes a) à e) pour chaque plan de découpe afin de déterminer une carte tridimensionnelle des valeurs minimales d'énergie nécessaire à la formation de bulles de gaz dans l'ensemble des zones élémentaires du tissu oculaire.

L'invention concerne également un procédé de détermination d'une valeur minimale d'énergie L.A.S.E.R. nécessaire pour former, en utilisant un appareil de découpe incluant une source L.A.S.E.R. femtoseconde, une bulle de gaz dans au moins une zone élémentaire d'un tissu oculaire, ***remarquable en ce que*** le procédé comprend les étapes suivantes :
a) Réception par une unité de traitement de données, d'une image de référence du tissu oculaire, ladite image de référence ayant été acquise par un système d'imagerie antérieurement à l'émission d'un faisceau L.A.S.E.R. apte à focaliser en un point d'échantillonnage respectif de chaque zone élémentaire,
b) Réception par l'unité de traitement de données, d'au moins une image courante du tissu oculaire, ladite image courante ayant été acquise par le système d'imagerie postérieurement à l'émission du faisceau L.A.S.E.R.,
c) Recherche par l'unité de traitement de données, d'au moins une bulle de gaz formée au niveau du point d'échantillonnage de chaque zone élémentaire, en comparant les images courante et de référence,
d) Assignation de l'énergie du faisceau L.A.S.E.R. à la valeur minimale associée à chaque zone élémentaire dans laquelle une bulle est détectée,
e) Augmentation de l'énergie du faisceau L.A.S.E.R. et réitération des étapes b) à e) pour chaque zone élémentaire dans laquelle aucune bulle n'est détectée.

Des aspects préférés mais non limitatifs du procédé selon l'invention sont les suivants :
- les images de référence et courante peuvent être des images OCT, l'étape de recherche comprenant les sous-étapes consistant à :
   - calculer au point d'échantillonnage, une variation d'intensité de lumière réfléchie reçue par le système d'imagerie entre les pixels de l'image courante et les pixels de l'image de référence,
   - comparer cette variation d'intensité calculée à une valeur seuil pour identifier la formation d'une bulle de gaz au point d'échantillonnage si la variation d'intensité calculée est supérieure à la valeur seuil ;
- le procédé peut comprendre en outre l'étape suivante :
   f) enregistrement par l'unité de traitement de données, de la valeur minimale associée à chaque zone élémentaire dans une mémoire
- le tissu oculaire peut comprendre un ensemble de zones élémentaires s'étendant dans une pluralité de plans de découpe contenant chacun au moins une zone élémentaire, le procédé comprenant la réitération des étapes a) à f) pour chaque plan de découpe afin de déterminer une carte tridimensionnelle des valeurs minimales d'énergie nécessaire à la formation de bulles de gaz dans l'ensemble des zones élémentaires du tissu oculaire.

L'invention concerne également un dispositif de détermination d'une valeur minimale d'énergie L.A.S.E.R. nécessaire pour former, en utilisant un appareil de découpe incluant une source L.A.S.E.R. femtoseconde, une bulle de gaz dans au moins une zone élémentaire d'un tissu oculaire, le dispositif de détermination incluant un système d'imagerie pour l'acquisition d'images, ***remarquable en ce que*** le dispositif de détermination comprend en outre une unité de traitement de données adaptée pour :
a) Recevoir une image de référence du tissu oculaire, ladite image de référence ayant été acquise par le système d'imagerie antérieurement à l'émission d'un faisceau L.A.S.E.R. apte à focaliser en un point d'échantillonnage respectif de chaque zone élémentaire,
b) Recevoir au moins une image courante du tissu oculaire, ladite image courante ayant été acquise par le système d'imagerie postérieurement à l'émission du faisceau L.A.S.E.R.,
c) Rechercher au moins une bulle de gaz formée au niveau du point d'échantillonnage de chaque zone élémentaire, en comparant les images courante et de référence,
d) Assigner l'énergie du faisceau L.A.S.E.R. à la valeur minimale associée à chaque zone élémentaire dans laquelle une bulle est détectée,
e) Augmenter l'énergie du faisceau L.A.S.E.R. et réitérer les étapes b) à e) pour chaque zone élémentaire dans laquelle aucune bulle n'est détectée.

Des aspects préférés mais non limitatifs du dispositif selon l'invention sont les suivants :
- les images de référence et courante peuvent être des images OCT, l'unité de traitement de données étant adaptée pour rechercher au moins une bulle de gaz en :
   - calculant au point d'échantillonnage, une variation d'intensité de lumière réfléchie reçue par le système d'imagerie entre les pixels de l'image courante et les pixels de l'image de référence,
   - comparant cette variation d'intensité calculée à une valeur seuil pour identifier la formation d'une bulle de gaz au point d'échantillonnage si la variation d'intensité calculée est supérieure à la valeur seuil ;
- l'unité de traitement de données peut en outre être adaptée pour :
   f) enregistrer la valeur minimale déterminée pour la zone élémentaire dans une mémoire ;
- le tissu oculaire peut comprendre un ensemble de zones élémentaires s'étendant dans une pluralité de plans de découpe contenant chacun au moins une zone élémentaire, l'unité de traitement de données étant en outre adaptée pour réitérer les étapes a) à f) pour chaque plan de découpe afin de déterminer une carte tridimensionnelle des valeurs minimales d'énergie nécessaire à la formation de bulles de gaz dans l'ensemble des zones élémentaires du tissu oculaire.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique d'un exemple d'appareil de découpe d'un tissu oculaire utilisant un laser femtoseconde ;
- la figure 2 est une représentation schématique d'un dispositif pour la détermination d'une valeur minimale d'énergie L.A.S.E.R. nécessaire à la formation d'une bulle de gaz dans un tissu oculaire ;
- la figure 3 est une représentation schématique d'une première variante de procédé pour la détermination d'une valeur minimale d'énergie L.A.S.E.R. nécessaire à la formation d'une bulle de gaz dans un tissu oculaire,
- la figure 4 est une représentation schématique d'une deuxième variante du procédé pour la détermination d'une valeur minimale d'énergie L.A.S.E.R. nécessaire à la formation d'une bulle de gaz dans le tissu oculaire,
- la figure 5 est une représentation schématique d'une troisième variante du procédé pour la détermination d'une valeur minimale d'énergie L.A.S.E.R. nécessaire à la formation d'une bulle de gaz dans le tissu oculaire.

### EXPOSE DETAILLE DE L'INVENTION

L'invention concerne un procédé et un dispositif de détermination d'une (ou plusieurs) valeur(s) minimale(s) d'énergie L.A.S.E.R. nécessaire à la formation d'une bulle de gaz dans une (ou plusieurs) zone(s) élémentaire(s) d'un tissu oculaire humain ou animal 60 au moyen d'un appareil de découpe incluant un laser femtoseconde.

Dans la suite de la description, l'invention sera décrite, à titre d'exemple, pour la découpe d'un cristallin, étant entendu que la présente invention peut s'appliquer à la détermination d'une (ou de) valeur(s) minimale(s) d'énergie nécessaire à la formation de bulles de gaz dans une (ou plusieurs) zone(s) élémentaire(s) d'autres tissus oculaires.

### 1. Appareil de découpe

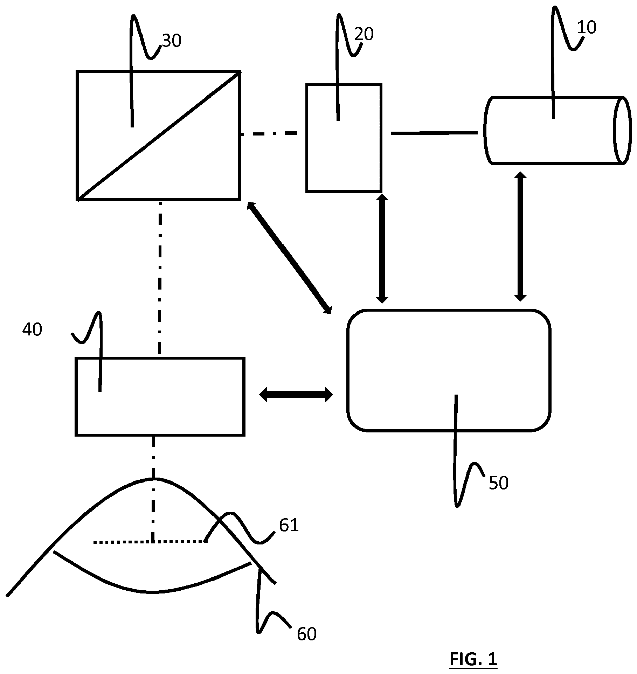

En référence à la figure 1, on a illustré un mode de réalisation de l'appareil de découpe selon l'invention. L'appareil de découpe comprend :
- un laser femtoseconde 10,
- un scanner optique de balayage 30 en aval du laser 10,
- un système optique de focalisation 40 en aval du scanner optique de balayage 30, et
- un système de commande 50 permettant de piloter le scanner optique de balayage 30 et le système optique de focalisation 40.

Le laser femtoseconde 10 est apte à émettre un faisceau L.A.S.E.R. sous la forme d'impulsions. A titre d'exemple, le laser 10 émet une lumière de 1030 nm de longueur d'onde, sous la forme d'impulsions de 400 femtosecondes. Le laser 10 possède une puissance de 20W et une fréquence de 500 kHz.

Le scanner optique de balayage 30 permet d'orienter le faisceau issu du laser 10 pour le déplacer le long d'un chemin de déplacement prédéfini par l'utilisateur dans un plan de focalisation 61.

Le système optique de focalisation 40 permet de focaliser le faisceau dans le plan de focalisation 61 - correspondant au plan de découpe.

L'appareil de découpe peut également inclure un système de mise en forme 20 - tel qu'un modulateur spatial de lumière à cristaux liquides (ou *« SLM* », acronyme de l'anglais « *Spatial Light Modulator* ») - entre le laser femtoseconde 10 et le scanner optique de balayage 30. Ce système de mise en forme 20 est positionné sur la trajectoire du faisceau issu du laser femtoseconde 10. Le système de mise en forme 20 permet de moduler la phase du faisceau issu du laser femtoseconde 10 pour répartir l'énergie du faisceau en une pluralité de points d'impact dans son plan focal, cette pluralité de points d'impact définissant un motif. Plus précisément, le système de mise en forme permet, à partir d'un faisceau L.A.S.E.R. gaussien générant un unique point d'impact, et au moyen du masque de phase, de répartir son énergie par modulation de phase de sorte à générer simultanément plusieurs points d'impact dans son plan de focalisation à partir d'un unique faisceau L.A.S.E.R. mis en forme par modulation de phase (un seul faisceau en amont et en aval du SLM). Cette génération d'une pluralité de points d'impact à partir d'un unique faisceau L.A.S.E.R. modulé permet (en plus d'une diminution du temps de découpe du tissu oculaire) d'améliorer la qualité de la découpe réalisée. En particulier, le système de mise en forme permet l'obtention d'un plan de découpe homogène, dans lequel les ponts tissulaires résiduels ont tous sensiblement les mêmes dimensions (en effet, même si une partie du faisceau LASER modulé est masqué, le nombre de points d'impact dans le plan de découpe reste identique). Cette amélioration de la qualité de la découpe facilite l'opération de dissection réalisée ultérieurement par le praticien.

Le système de commande 50 permettant de piloter le scanner optique de balayage 30, l'éventuel système de mise en forme 20 et le système optique de focalisation 40. Le système de commande 50 peut être composée d'une (ou plusieurs) station(s) de travail, et/ou d'un (ou plusieurs) ordinateur(s) ou peut être de tout autre type connu de l'homme du métier. Le système de commande 50 peut par exemple comprendre un téléphone portable, une tablette électronique (tel qu'un IPAD^{®}), un assistant personnel (ou « *PDA* », sigle de l'expression anglo-saxonne « *Personal Digital Assistant* »), etc. Dans tous les cas, le système de commande 50 comprend un processeur programmé pour permettre le pilotage du laser femtoseconde 10, le scanner optique de balayage 30, le système optique de focalisation 40, le système de mise en forme 20, etc.

### 2. Dispositif de détermination

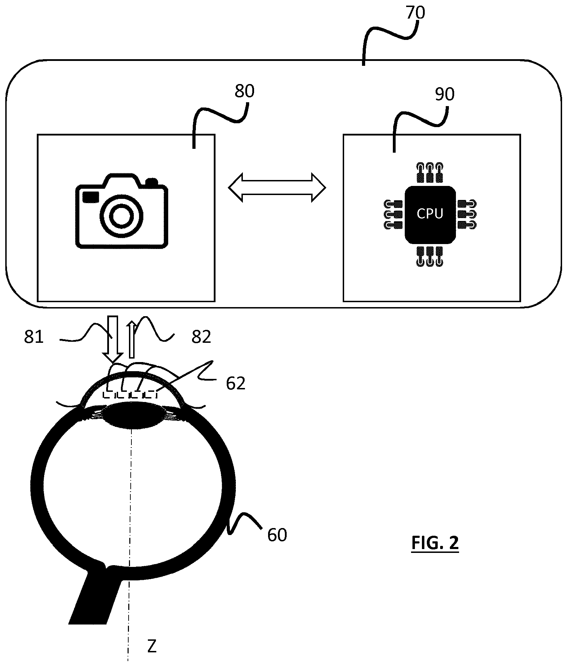

En référence à la figure 2, le dispositif de détermination 70 comprend :
- un système d'imagerie 80 pour l'acquisition d'images de type OCT (signifiant « *Optical Cohérence Tomography »,* ou tomographie par cohérence optique), ou de type Scheimpflug (cartographie en lumière visible), ou de type UBM (signifiant *« Ultrasonic Bio Microscopy »,* ou biomicroscopie ultrasonore),
- une unité de traitement 90 - incluant par exemple un processeur et une mémoire - pour traiter les images acquises par le système d'acquisition 80 et pour déterminer une carte (bi ou tridimensionnelle) des valeurs minimales d'énergie laser nécessaires à la formation de bulles de gaz dans une pluralité de zones élémentaires 62 du tissu oculaire 60.

Le dispositif de détermination 70 peut être intégré à l'appareil de découpe.

Notamment dans un mode de réalisation préféré :
- l'unité de traitement 90 fait partie du système de commande 50, et
- le système d'acquisition 80, le système de mise en forme 20, le scanner optique de balayage 30 et le système optique de focalisation 40 sont montés dans un compartiment fixé à l'extrémité d'un bras robotisé, tandis que le laser femtoseconde 10 peut être intégré à un caisson sur lequel est fixé le bras robotisé.

En variante, le dispositif de détermination 70 peut être distant de l'appareil de découpe et comprendre des moyens de communication avec ou sans fil (non représentés) pour l'échange d'informations entre le dispositif de détermination 70 et l'appareil de découpe.

Dans tous les cas, le dispositif de détermination 70 est programmé pour mettre en oeuvre le procédé de détermination décrit dans la suite.

### 3. Procédé de détermination

### 3.1. Généralité sur le procédé

Le procédé comprend les étapes suivantes :
- Acquisition d'une image de référence du tissu oculaire à traiter,
- Emission dans au moins une zone élémentaire d'au moins deux faisceaux L.A.S.E.R. d'énergies différentes,
- Acquisition d'au moins une image courante du tissu oculaire,
- Comparaison de l'image courante à l'image de référence pour détecter au moins une bulle de gaz associée à l'un des faisceaux L.A.S.E.R.,
- Déterminer une valeur minimale d'énergie nécessaire à la formation d'une bulle de gaz dans la zone élémentaire.

Chaque zone élémentaire 62 peut consister en une ligne (par exemple de 50 ou 100 µm), une surface (de 50×50µm ou de 100×100µm ou de 1000×1000µm) ou un volume élémentaire (de 50×50×50µm ou de 100×100×100µm ou de 1000×1000×1000µm), la combinaison des zones élémentaires permettant de définir une ligne totale, une surface totale ou un volume total à découper.

### 3.1.1. Images acquises

L'image de référence et l'image (ou les images) courante(s) acquises par le système d'imagerie peuvent être de type OCT, ou de type Scheimpflug, ou de type UBM.

L'un des avantages liés à l'acquisition d'images OCT concerne le fait qu'un système d'imagerie 80 de type OCT présente une haute sensibilité (≈ 100dB) permettant l'obtention d'image de haute qualité. En outre l'appareil de découpe intègre un système d'imagerie de type OCT, de sorte qu'aucun système d'imagerie supplémentaire n'est nécessaire. Enfin, l'image de référence de type OCT peut également être utilisée pour vérifier l'alignement de l'appareil de découpe avec le tissu oculaire 60 à traiter.

Pour l'acquisition d'une image OCT, un faisceau optique 81 est dirigé vers le tissu oculaire 60, et une petite portion de la lumière 82 qui est rétrodiffusée (par les différentes couches) du tissu oculaire 60 est recombinée sur un (ou plusieurs) capteur(s) du système d'imagerie 80 avec un signal de référence. Le signal enregistré par le (ou les) capteur(s) est modulé selon la différence de chemin optique entre le signal rétrodiffusé par le tissu oculaire 60 et le signal de référence. Ce signal enregistré est utilisé pour construire l'image OCT, qui peut être une image monodimensionnelle ou une image bidimensionnelle.

Plus précisément, le système d'imagerie 80 de type OCT peut être utilisé :
- en « *mode A* » (aussi appelé « A-scan »), ou
- en « *mode B »* (aussi appelé « B-scan »).

Le « *mode A* » permet l'acquisition d'une image OCT monodimensionnelle. Il est basé sur l'émission d'une information lumineuse 81 (par un émetteur du système d'imagerie) et la réception de l'interférence entre une lumière rétrodiffusée 82 (par le capteur du système d'imagerie) le long d'une ligne de propagation et la lumière de référence : l'image OCT monodimensionnelle obtenue (à partir du signal enregistré par le capteur) est représentative du profil de rétrodiffusion axial (i.e. en profondeur Z) du tissu oculaire 60 au point considéré (de coordonnée X, Y dans le plan de focalisation 61).

Le « *mode B »* permet l'acquisition d'une image OCT bidimensionnelle, en mettant en oeuvre un balayage transversal du tissu oculaire 60, par exemple selon une direction X perpendiculaire à l'axe optique Z du tissu oculaire 60. On obtient ainsi une pluralité de profils de rétrodiffusion en « *mode A »* établis pour différents points du tissu oculaire 60 selon la direction X. L'empilement de ces profils de rétrodiffusion en « *mode A* » obtenus permet de construire une image OCT en deux dimensions (XZ).

### 3.1.2. Etape de comparaison

L'étape de comparaison permet de détecter la formation éventuelle d'une bulle de gaz. En effet, les propriétés de rétrodiffusion de la lumière par le tissu oculaire 60 sont la source de contraste des images d'OCT, révélatrices d'informations morphologiques. Lorsqu'une bulle de gaz se forme dans le tissu oculaire 60, celle-ci engendre une variation des propriétés de rétrodiffusion de la lumière par le tissu oculaire 60.

L'étape de comparaison repose sur la détection de la variation d'intensité de la lumière rétrodiffusée reçue par le système d'imagerie 80 entre les pixels de l'image courante et les pixels de l'image de référence. Si cette variation d'intensité est supérieure à une valeur seuil préalablement définie, alors elle est représentative de la formation d'une bulle de gaz au point d'impact du faisceau L.A.S.E.R. considéré. Cela signifie que l'énergie du faisceau L.A.S.E.R. était suffisante pour former une bulle de gaz dans la zone élémentaire 62.

Afin d'assurer une découpe efficace du cristallin, la valeur minimale déterminée peut être rendue égale à multiple - compris entre 1 et 2 (par exemple 1.5x ou 2x) - de l'énergie du faisceau LA.S.E.R. ayant permis de former la bulle de gaz détectée. On garantit alors que la valeur minimale d'énergie est suffisante pour obtenir l'effet de découpe attendu et :
- qu'il est inutile d'utiliser un niveau d'énergie plus élevé que la valeur minimale déterminée, le surcroît d'énergie inutile pouvant nuire à la sécurité du patient,
- qu'un niveau d'énergie inférieur aboutirait à un défaut d'efficacité, l'énergie disponible étant insuffisante pour obtenir la formation d'une bulle de gaz.

Différents modes de réalisation du procédé peuvent être envisagés. Notamment, dans des premier et troisième modes de réalisation, les faisceaux L.A.S.E.R. d'énergies différentes sont émis en un unique point d'échantillonnage dans chaque zone élémentaire. Dans un deuxième mode de réalisation, chaque faisceau L.A.S.E.R. d'énergie différente est émis en un point distinct de la zone élémentaire, formant ainsi une série de points adjacents.

Ces différents modes de réalisation et leurs avantages associés sont décrits dans la suite.

### 3.2. Premier mode de réalisation du procédé

Dans le premier mode de réalisation, les faisceaux L.A.S.E.R. sont émis en un unique point d'échantillonnage pour chaque zone élémentaire 62 du tissu oculaire 60.

Pour une zone élémentaire 62 considérée, les faisceaux L.A.S.E.R. d'énergies différentes - en particulier croissante - sont émis successivement au niveau du point d'échantillonnage.

Une image courante est acquise après chaque émission d'un faisceau L.A.S.E.R. Avantageusement lorsque le tissu comprend plusieurs zones élémentaires s'étendant dans un même plan, une image courante peut être acquise après chaque émission d'un faisceau L.A.S.E.R. en un point d'échantillonnage respectif de chaque zone élémentaire. Ceci permet de limiter le nombre d'images courantes acquises, et ainsi d'augmenter la vitesse du procédé selon l'invention en limitant le temps nécessaire à l'acquisition des images courantes.

Après chaque acquisition d'une image courante, celle-ci est comparée à l'image de référence pour détecter si une bulle de gaz s'est formée.

Si une bulle s'est formée, alors l'énergie du faisceau L.A.S.E.R. est suffisante pour former une bulle de gaz : cette valeur d'énergie suffisante est enregistrée dans une mémoire et une nouvelle zone élémentaire 62 est étudiée pour déterminer la valeur d'énergie minimale nécessaire à la formation d'une bulle de gaz dans cette nouvelle zone élémentaire 62.

Plus précisément, selon ce premier mode de réalisation :
- un premier faisceau L.A.S.E.R. ayant une première énergie est émis pour focaliser au point d'échantillonnage de la zone élémentaire 62 considérée ; une première image courante est acquise et comparée à l'image de référence pour détecter la formation éventuelle d'une bulle de gaz ; si une bulle de gaz s'est formée, alors la première énergie est suffisante pour former la bulle de gaz : cette première énergie correspond à la valeur minimale d'énergie nécessaire à la formation d'une bulle de gaz dans la zone élémentaire 62 considérée; une nouvelle zone élémentaire est ensuite étudiée,
- sinon un deuxième faisceau L.A.S.E.R. ayant une deuxième énergie supérieure à la première énergie est émis au point d'échantillonnage et une deuxième image courante est acquise pour détecter la formation d'une éventuelle bulle de gaz ; si une bulle de gaz s'est formée, alors la deuxième énergie correspond à la valeur minimale d'énergie nécessaire à la formation d'une bulle de gaz dans la zone élémentaire considérée ; une nouvelle zone élémentaire 62 est ensuite étudiée,
- sinon un troisième faisceau L.A.S.E.R. ayant une troisième énergie supérieure à la deuxième énergie est émis ; une troisième image courante est acquise pour détecter la formation d'une éventuelle bulle de gaz ; si une bulle de gaz s'est formée, alors la troisième énergie correspond à la valeur minimale d'énergie nécessaire à la formation d'une bulle de gaz dans la zone élémentaire 62 considérée ; une nouvelle zone élémentaire 62 est ensuite étudiée,
- etc. jusqu'à détermination de la valeur minimale d'énergie nécessaire à la formation d'une bulle de gaz dans la zone élémentaire considérée.

En variante lorsqu'une unique image courante est acquise postérieurement à l'émission d'un faisceau L.A.S.E.R. en un point d'échantillonnage respectif de plusieurs zones élémentaires s'étendant dans un même plan :
- un premier faisceau L.A.S.E.R. est émis pour focaliser au point d'échantillonnage de chaque zone élémentaire s'étendant dans un même plan ; une première image courante est acquise et comparée à l'image de référence pour détecter la formation éventuelle d'une bulle de gaz dans l'une (ou dans plusieurs) des zones élémentaires ; si une bulle de gaz s'est formée dans l'une (ou dans plusieurs) de ces zones, la valeur minimale d'énergie est déterminée pour cette (ou ces) zone(s),
- si certaines zones ne contiennent pas de bulle de gaz, un deuxième faisceau L.A.S.E.R. d'énergie supérieure est émis au point d'échantillonnage ce ces certaines zones et une deuxième image courante est acquise pour détecter la formation d'une éventuelle bulle de gaz ;
- etc. jusqu'à détermination des valeurs minimales dans toutes les zones élémentaires s'étendant dans un même plan.

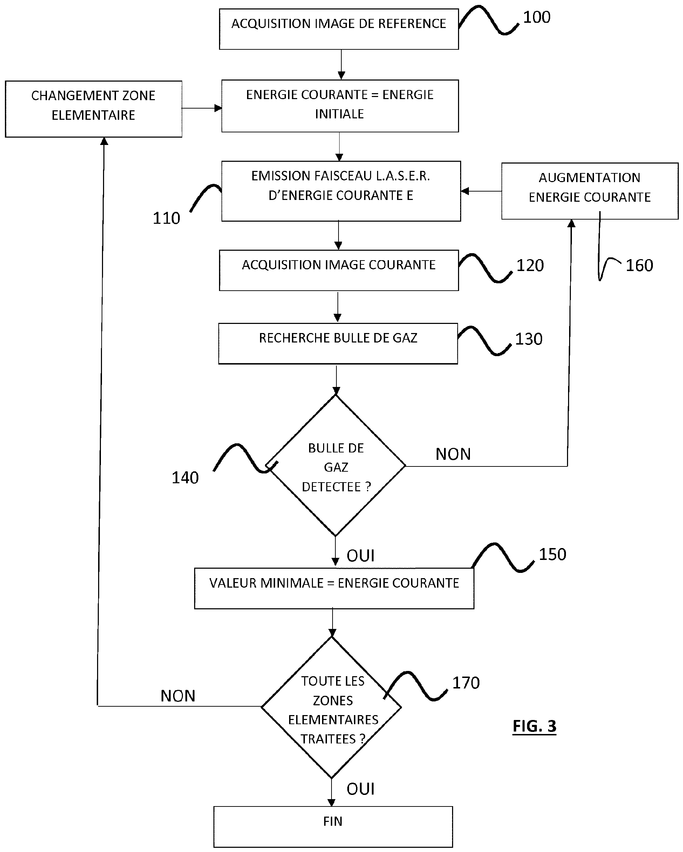

Ainsi et comme illustré à la figure 3, le procédé comprend, pour chaque zone élémentaire 62 :
a) acquisition 100 d'une image de référence,
b) émission 110 sur le point d'échantillonnage de chaque zone élémentaire, d'un faisceau L.A.S.E.R. d'énergie comprise entre une énergie minimum Eₘᵢₙ (supposée suffisante a priori pour générer une bulle de gaz dans le tissu) et une énergie maximum Eₘₐₓ (un multiple de Eₘᵢₙ (par exemple Eₘₐₓ₌ 10 × Eₘᵢₙ)),
c) acquisition 120 d'une image courante du tissu oculaire, ladite image courante contenant le point d'échantillonnage de chaque zone élémentaire,
d) recherche 130 d'une bulle de gaz dans l'image courante en déterminant une variation d'intensité de la lumière rétrodiffusée entre l'image de référence et l'image courante (par exemple par soustraction de l'image de référence à l'image courante) pour chaque zone élémentaire,
e) comparaison 140 de la variation d'énergie déterminée dans chaque zone élémentaire à une valeur seuil,
f) pour chaque zone élémentaire, enregistrement 150 de la valeur d'énergie du faisceau L.A.S.E.R. et de la position de la zone élémentaire dans une mémoire si la variation d'énergie déterminée est supérieure à la valeur seuil,
g) augmentation 160 de l'énergie du faisceau L.A.S.E.R. et réitération des étapes b) à g) pour chaque zone élémentaire dans laquelle aucune bulle n'est détectée.

Une fois l'ensemble des zones élémentaires 62 traitées, on obtient une cartographie (notamment tridimensionnelle) des valeurs minimales d'énergie nécessaires à la formation de bulles de gaz dans chacune des zones élémentaires 62 du tissu oculaire 60.

Un avantage de ce premier mode de réalisation concerne le fait que les faisceaux L.A.S.E.R. d'énergies croissantes sont émis en un unique point d'échantillonnage pour chaque zone élémentaire 62, sans émission d'un faisceau L.A.S.E.R. d'énergie supérieure à la valeur minimale d'énergie nécessaire à la formation d'une bulle de gaz. Toutefois, ce premier mode de réalisation est coûteux en temps, du fait de l'acquisition d'une pluralité d'images courantes.

C'est pourquoi les inventeurs ont proposé un deuxième mode de réalisation du procédé dans lequel le nombre d'images courantes acquises est limité à une image par zone élémentaire 62, voire une image pour l'ensemble des zones élémentaires 62.

### 3.3. Deuxième mode de réalisation du procédé

Dans le deuxième mode de réalisation, les faisceaux L.A.S.E.R. sont émis en une pluralité de points d'échantillonnage pour chaque zone élémentaire 62 du tissu oculaire 60, par exemple en huit points régulièrement espacés de 80µm sur une ligne de 560µm. Plus précisément pour chaque zone élémentaire 62, chaque faisceau L.A.S.E.R. d'énergie croissante est émis (simultanément ou successivement) en un point d'échantillonnage respectif.

Une image courante est acquise pour chaque zone élémentaire 62, une fois tous les faisceaux L.A.S.E.R. d'énergies différentes émis dans ladite zone élémentaire 62.

L'image courante est comparée à l'image de référence pour détecter si une (ou plusieurs) bulle(s) de gaz s'est (se sont) formée(s). Si plusieurs bulles de gaz sont formées, alors l'énergie la plus faible ayant permis l'obtention d'une bulle de gaz est sélectionnée en tant que valeur minimale d'énergie nécessaire à la formation d'une bulle de gaz.

Plus précisément, selon ce deuxième mode de réalisation, des premier, deuxième, troisième (...etc.) faisceaux L.A.S.E.R. ayant des première, deuxième, troisième énergies croissantes (i.e. première énergie < deuxième énergie < troisième énergie) sont émis pour focaliser en trois points d'échantillonnage respectifs de la zone élémentaire 62 considérée. Une image courante est acquise et comparée à l'image de référence pour détecter la formation éventuelle de bulles de gaz :
- si une seule bulle de gaz s'est formée au niveau du point d'échantillonnage associé au troisième faisceau L.A.S.E.R., alors la troisième énergie est suffisante pour former la bulle de gaz : cette troisième énergie correspond à la valeur minimale d'énergie nécessaire à la formation d'une bulle de gaz dans la zone élémentaire 62 considérée ; une nouvelle zone élémentaire 62 est ensuite étudiée,
- si plusieurs bulles de gaz sont formées, par exemple pour les deuxième et troisième faisceaux, alors la deuxième énergie (qui est inférieure à la troisième énergie) correspond à la valeur minimale d'énergie nécessaire à la formation d'une bulle de gaz dans la zone élémentaire 62 considérée ; une nouvelle zone élémentaire 62 est ensuite étudiée.

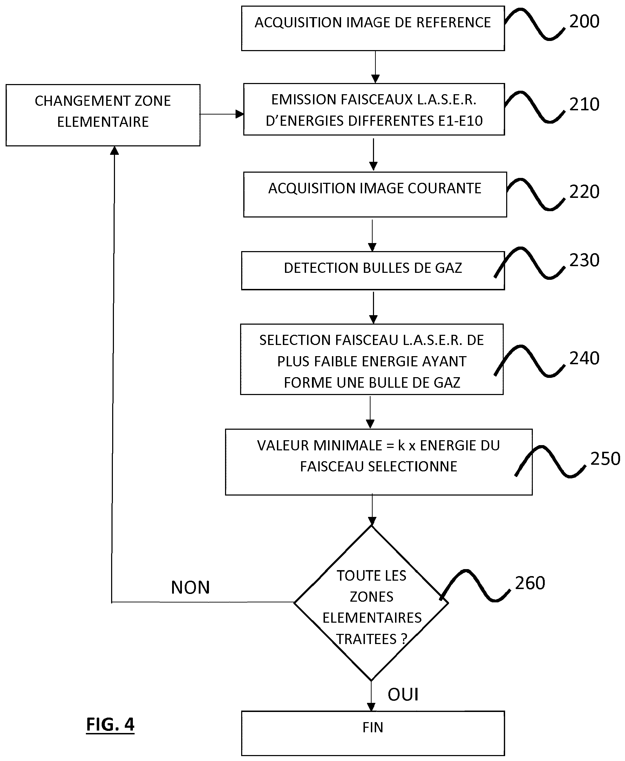

Ainsi et comme illustré à la figure 4, le procédé comprend, pour chaque zone élémentaire :
a) acquisition 200 d'une image de référence,
b) émission 210 sur une pluralité de points d'échantillonnage respectifs, d'une pluralité de faisceaux L.A.S.E.R. d'énergies différentes comprises entre l'énergie minimum Eₘᵢₙ et l'énergie maximum Eₘₐₓ,
c) acquisition 220 d'une image courante du tissu oculaire, ladite image courante contenant la pluralité de points d'échantillonnage,
d) détection 230 d'une (ou plusieurs) bulle(s) de gaz par comparaison de l'image courante à l'image de référence,
e) sélection 240 de la bulle de gaz associée au faisceau L.A.S.E.R. d'énergie la plus faible parmi l'ensemble des bulles de gaz,
f) enregistrement 250 de la valeur d'énergie du faisceau L.A.S.E.R. associé à la bulle de gaz sélectionnée (et de la position de la zone élémentaire) dans une mémoire,
g) si toutes les zones élémentaires n'ont pas été traitées, réitération des étapes b) à g) pour une nouvelle zone élémentaire 62.

Bien entendu, il est également possible dans ce mode de réalisation d'acquérir une seule image courante pour plusieurs zones élémentaires simultanément (si ces zones élémentaires se situent dans un même plan Bscan). Dans ce cas, les étapes de traitement (détection de bulle et détermination de la valeur minimale d'énergie pour chaque zone élémentaire) peuvent être mises en oeuvre pour ces plusieurs zones élémentaires à partir d'une seule image courante afin d'économiser du temps.

Une fois l'ensemble des zones élémentaires 62 traitées, on obtient une cartographie (notamment tridimensionnelle) des valeurs minimales d'énergie nécessaires à la formation de bulles de gaz dans chacune des zones élémentaires du tissu oculaire.

Comme indiqué précédemment, un avantage de ce deuxième mode de réalisation concerne le fait que la détermination des valeurs minimales d'énergie nécessaires à la formation de bulles de gaz dans chaque zone élémentaire 62 est plus rapide qu'avec le premier mode de réalisation étant donné qu'il est nécessaire de ne réaliser qu'une seule acquisition d'image courante par zone élémentaire étudiée, voir une seule acquisition d'image courante pour plusieurs zones élémentaires s'étendant dans un même plan.

### 3.4. Troisième mode de réalisation du procédé

Le troisième mode de réalisation du procédé peut être considéré comme une combinaison des premier et deuxième modes de réalisation.

Plus précisément dans ce premier mode de réalisation, les faisceaux L.A.S.E.R. d'énergies différentes - en particulier croissante - sont émis successivement en un unique point d'échantillonnage pour chaque zone élémentaire 62 du tissu oculaire 60. Une image courante est acquise après chaque émission d'un faisceau L.A.S.E.R. sur un point d'échantillonnage respectif de plusieurs zones élémentaires s'étendant dans un même plan.

Par exemple huit faisceaux L.A.S.E.R. d'énergies E₁ à E₈ sont émis successivement en un point d'échantillonnage respectif de chaque zone élémentaire s'étendant dans le même plan.

Une première image courante est acquise après émission du faisceau d'énergie E₁ au point d'échantillonnage respectif de chaque zone élémentaire s'étendant dans le même plan. Une deuxième image courante est acquise après émission du faisceau d'énergie E₂ au point d'échantillonnage respectif de chaque zone élémentaire s'étendant dans le même plan, et ainsi de suite jusqu'à l'acquisition d'une huitième image courante après émission du faisceau d'énergie E₈ au point d'échantillonnage respectif de chaque zone élémentaire s'étendant dans le même plan.

Les huit images courantes sont ensuite comparées à l'image de référence pour détecter la présence d'une bulle de gaz dans chaque zone élémentaire. Lorsque plusieurs bulles de gaz sont détectées pour une même zone élémentaire à partir de plusieurs images courantes (par exemple une bulle de gaz est détectée à partir des sixième, septième et huitième images courantes pour une zone considérée), alors l'énergie la plus faible parmi les énergies des faisceaux L.A.S.E.R. ayant induit la génération d'une bulle de gaz est utilisée pour déterminer la valeur minimale nécessaire à la formation d'une bulle de gaz.

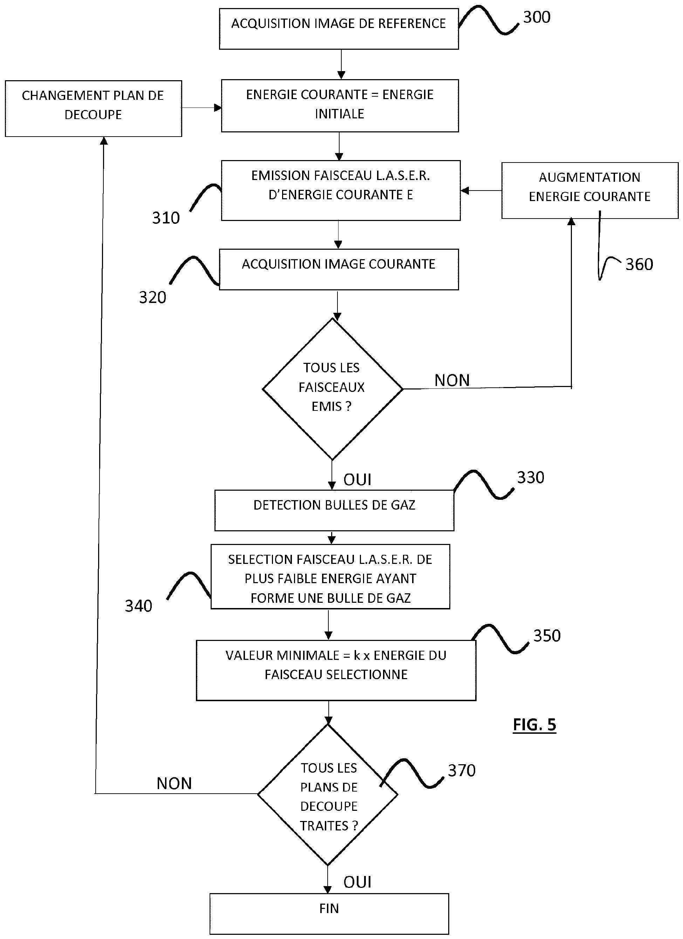

Ainsi et comme illustré à la figure 5, le procédé comprend :
a) acquisition 300 d'une image de référence,
b) émission 310 en un point d'échantillonnage respectif de chaque zone élémentaire s'étendant dans un même plan, d'un faisceau L.A.S.E.R. d'énergie comprise entre une énergie minimum Eₘᵢₙ et une énergie maximum Eₘₐₓ,
c) acquisition 320 d'une image courante du tissu oculaire, ladite image courante contenant les points d'échantillonnage des zones élémentaires s'étendant dans le même plan,
d) augmentation 360 de l'énergie du faisceau L.A.S.E.R. d'une valeur prédéterminée et réitération des étapes b) à d) si l'énergie augmentée est inférieure à l'énergie maximum, ou passage à l'étape e) si l'énergie augmentée est supérieure à l'énergie maximum
e) pour chaque zone élémentaire s'étendant dans le même plan, détection 330 d'une (ou plusieurs) bulle(s) de gaz par comparaison des images courantes à l'image de référence,
f) pour chaque zone élémentaire s'étendant dans le même plan, sélection 340 de la bulle de gaz associée au faisceau L.A.S.E.R. d'énergie la plus faible parmi l'ensemble des bulles de gaz, et détermination 350 de la valeur minimale d'énergie,
g) pour chaque zone élémentaire s'étendant dans le même plan, enregistrement de la valeur d'énergie déterminée (et de la position de la zone élémentaire) dans une mémoire,
h) réitération 370 des étapes a) à g) pour de nouvelles zones élémentaires 62 s'étendant dans un autre plan si besoin.

Dans ce mode de réalisation permet de limiter la durée nécessaire au procédé pour déterminer les valeurs minimales associées aux différentes zones élémentaires du tissu oculaire. En effet, les étapes relatives au traitement des images courantes peuvent être mise en oeuvre durant le pilotage du système optique de focalisation 40 pour déplacer le plan de focalisation 61 d'un plan de découpe à un autre.

Par ailleurs, le fait de continuer à augmenter l'énergie du faisceau L.A.S.E.R. émis sur le point d'échantillonnage de chaque zone élémentaire même si une bulle de gaz s'est déjà formée permet de limiter les risques de « faux-positifs » lors de la détection (si une bulle de gaz est détectée dans une courante acquise postérieurement à l'émission d'un faisceau L.A.S.E.R. d'énergie donnée alors qu'elle est absente de l'image courante acquise postérieurement à l'émission d'un faisceau L.A.S.E.R. d'énergie supérieure à l'énergie donnée : alors cette bulle de gaz détectée constitue un faux positif).

### 4. Conclusions

Ainsi, l'invention permet la détermination une valeur minimale d'énergie L.A.S.E.R. suffisante pour former une bulle de gaz dans au moins une zone élémentaire d'un tissu oculaire au moyen d'un appareil de découpe incluant une source L.A.S.E.R. femtoseconde. Ceci permet de limiter la quantité d'énergie excédentaire du faisceau susceptible de se propager jusqu'à la rétine afin de réduire l'échauffement local de la rétine lors de la mise en oeuvre de l'opération de découpe du tissu oculaire au moyen du dispositif de découpe. Ceci permet également de s'assurer à contrario, que la quantité d'énergie envoyée est suffisante pour obtenir l'effet attendu et éviter ainsi les problèmes liés au manque d'efficacité lors de la découpe du tissu avec un tissu seulement partiellement découpé.

L'invention est définie par les revendications suivantes.

## Revendications

1. Dispositif (70) de détermination d'une valeur minimale d'énergie L.A.S.E.R. nécessaire pour former, en utilisant un appareil de découpe incluant une source L.A.S.E.R. femtoseconde (10), une bulle de gaz dans au moins une zone élémentaire (62) s'étendant dans un plan de découpe d'un tissu oculaire (60), le dispositif de détermination (70) incluant un système d'imagerie (80) pour l'acquisition d'images, le dispositif de détermination (70) comprenant en outre une unité de traitement de données (90) incluant des moyens pour :
a) Recevoir une image de référence du tissu oculaire, ladite image de référence ayant été acquise (200, 300) par le système d'imagerie (80) antérieurement à l'émission (210, 310) d'une pluralité de faisceaux L.A.S.E.R. aptes à focaliser en au moins un point d'échantillonnage de chaque zone élémentaire (62), chaque faisceau L.A.S.E.R. ayant une énergie respective différente des autres faisceaux L.A.S.E.R. de la pluralité,
b) Recevoir au moins une image courante du tissu oculaire (60), chaque image courante ayant été acquise (220, 320) par le système d'imagerie (80) postérieurement à l'émission (210, 310) d'au moins un faisceau L.A.S.E.R. de la pluralité de faisceaux L.A.S.E.R.,
puis pour chaque zone élémentaire :
c) Détecter en comparant l'image de référence à chaque image courante, au moins une bulle de gaz formée dans la zone élémentaire, chaque bulle de gaz étant associée à un faisceau L.A.S.E.R. respectif,
d) Déterminer en fonction de l'au moins une bulle de gaz, une valeur minimale d'énergie nécessaire à la formation d'une bulle de gaz dans la zone élémentaire.

2. Dispositif de détermination selon la revendication 1, ***dans lequel*** les images de référence et courante(s) sont des images OCT, les moyens pour détecter étant, pour chaque image courante, aptes à :
• calculer en chaque point d'échantillonnage, une variation d'intensité de lumière rétrodiffusée reçue par le système d'imagerie (80) entre les pixels de l'image courante et les pixels de l'image de référence,
• comparer cette variation d'intensité calculée à une valeur seuil pour identifier la formation d'une bulle de gaz si la variation d'intensité calculée est supérieure à la valeur seuil.

3. Dispositif de détermination selon l'une quelconque des revendications 1 ou 2, dans lequel pour chaque zone élémentaire, chaque faisceau L.A.S.E.R. est apte à focaliser en un unique point d'échantillonnage de la zone élémentaire, les moyens pour recevoir étant aptes à recevoir une pluralité d'images courantes du tissu oculaire, chaque image courante ayant été acquise par le système d'imagerie postérieurement à l'émission d'un faisceau L.A.S.E.R. respectif dans chaque zone élémentaire.

4. Dispositif de détermination selon la revendication 3, ***dans lequel*** les moyens pour déterminer sont, pour chaque zone élémentaire, aptes à :
- si une unique bulle de gaz est détectée :
• assigner à la valeur minimale, un multiple k de l'énergie du faisceau L.A.S.E.R. ayant induit la formation de la bulle de gaz détectée, k étant un nombre décimal compris entre 1 et 2,
- si plusieurs bulles de gaz sont détectées :
• sélectionner parmi les images courantes dans lesquelles une bulle de gaz est détectée, l'image courante associée au faisceau L.A.S.E.R. d'énergie la plus faible, et
• assigner à la valeur minimale, un multiple k de l'énergie du faisceau L.A.S.E.R. associé à l'image courante sélectionnée, k étant un nombre décimal compris entre 1 et 2.

5. Dispositif de détermination selon l'une quelconque des revendications 1 ou 2, ***dans lequel*** pour chaque zone élémentaire, chaque faisceau L.A.S.E.R. est apte à focaliser en un point d'échantillonnage respectif de la zone élémentaire, les moyens pour recevoir étant aptes à recevoir une image courante du tissu oculaire, ladite image courante ayant été acquise par le système d'imagerie postérieurement à l'émission de la pluralité de faisceaux L.A.S.E.R. en un point d'échantillonnage respectif de chaque zone élémentaire.

6. Dispositif de détermination selon la revendication 5, ***dans lequel*** les moyens pour déterminer sont, pour chaque zone élémentaire, aptes à :
- si une unique bulle de gaz est détectée :
• assigner à la valeur minimale, un multiple k de l'énergie du faisceau L.A.S.E.R. associé au point d'échantillonnage au niveau duquel la bulle de gaz est détectée, k étant un nombre décimal compris entre 1 et 2,
- si plusieurs bulles de gaz sont détectées :
• sélectionner parmi les points d'échantillonnage au niveau de chacun desquels une bulle de gaz est détectée, le point d'échantillonnage associé au faisceau L.A.S.E.R. d'énergie la plus faible, et
• assigner à la valeur minimale, un multiple k de l'énergie du faisceau L.A.S.E.R. associé audit point d'échantillonnage sélectionné, k étant un nombre décimal compris entre 1 et 2.

7. Dispositif de détermination selon l'une quelconque des revendications 1 à 6, ***dans lequel*** l'unité de traitement comprend en outre des moyens pour :
e) enregistrer la valeur minimale déterminée pour chaque zone élémentaire (62) dans une mémoire.

8. Dispositif selon la revendication 7, ***dans lequel*** le tissu oculaire comprend un ensemble de zones élémentaires s'étendant dans une pluralité de plans de découpe contenant chacun au moins une zone élémentaire (62), l'unité de traitement de données étant en outre adaptée pour réitérer les étapes a) à e) pour chaque plan de découpe afin de déterminer une carte tridimensionnelle des valeurs minimales d'énergie nécessaire à la formation de bulles de gaz dans l'ensemble des zones élémentaires (62) du tissu oculaire (60).

9. Procédé de détermination d'une valeur minimale d'énergie L.A.S.E.R. nécessaire pour former, en utilisant un appareil de découpe incluant une source L.A.S.E.R. femtoseconde (10), une bulle de gaz dans au moins une zone élémentaire (62) s'étendant dans un plan de découpe d'un tissu oculaire (60), le procédé comprenant les étapes suivantes :
a) Réception par une unité de traitement de données (90), d'une image de référence du tissu oculaire (60), ladite image de référence ayant été acquise (200, 300) par un système d'imagerie (80) antérieurement à l'émission (210, 310) d'une pluralité de faisceaux L.A.S.E.R. aptes à focaliser en au moins un point d'échantillonnage de chaque zone élémentaire (62), chaque faisceau L.A.S.E.R. ayant une énergie respective différente des autres faisceaux L.A.S.E.R. de la pluralité de faisceaux L.A.S.E.R.,
b) Réception par l'unité de traitement de données (90), d'au moins une image courante du tissu oculaire (60), chaque image courante ayant été acquise (220, 320) par le système d'imagerie (80) postérieurement à l'émission (210, 310) d'au moins un faisceau L.A.S.E.R. de la pluralité de faisceaux L.A.S.E.R.,
puis pour chaque zone élémentaire, mise en oeuvre des étapes suivantes par l'unité de traitement de données (90) :
c) Détection (230, 330) en comparant l'image de référence à chaque image courante, d'au moins une bulle de gaz formée dans la zone élémentaire, chaque bulle de gaz étant associée à un faisceau L.A.S.E.R. respectif,
d) Détermination (240, 250 ; 340, 350) en fonction de l'au moins une bulle de gaz détectée, d'une valeur minimale d'énergie nécessaire à la formation d'une bulle de gaz dans la zone élémentaire.

10. Procédé de détermination selon la revendication 9, ***dans lequel*** les images de référence et courante(s) sont des images OCT, l'étape de détection (230, 330) comprenant, pour chaque image courante, les sous-étapes consistant à :
• calculer en chaque point d'échantillonnage, une variation d'intensité de lumière rétrodiffusée reçue par le système d'imagerie (80) entre les pixels de l'image courante et les pixels de l'image de référence,
• comparer cette variation d'intensité calculée à une valeur seuil pour identifier la formation d'une bulle de gaz si la variation d'intensité calculée est supérieure à la valeur seuil.

11. Procédé de détermination selon l'une quelconque des revendications 9 ou 10, dans lequel pour chaque zone élémentaire, chaque faisceau L.A.S.E.R. est apte à focaliser en un unique point d'échantillonnage de la zone élémentaire, l'étape b) de réception consistant à recevoir une pluralité d'images courantes du tissu oculaire, chaque image courante ayant été acquise par le système d'imagerie postérieurement à l'émission d'un faisceau L.A.S.E.R. respectif dans chaque zone élémentaire.

12. Procédé de détermination selon la revendication 11, dans lequel l'étape de détermination inclut les sous-étapes suivantes pour chaque zone élémentaire :
- si une unique bulle de gaz est détectée :
• assigner à la valeur minimale, un multiple k de l'énergie du faisceau L.A.S.E.R. ayant induit la formation de la bulle de gaz détectée, k étant un nombre décimal compris entre 1 et 2,
- si plusieurs bulles de gaz sont détectées :
• sélectionner parmi les images courantes dans lesquelles une bulle de gaz est détectée, l'image courante associée au faisceau L.A.S.E.R. d'énergie la plus faible, et
• assigner à la valeur minimale, un multiple k de l'énergie du faisceau L.A.S.E.R. associé à l'image courante sélectionnée, k étant un nombre décimal compris entre 1 et 2.

13. Procédé de détermination selon l'une quelconque des revendications 9 ou 10, dans lequel pour chaque zone élémentaire, chaque faisceau L.A.S.E.R. est apte à focaliser en un point d'échantillonnage respectif de la zone élémentaire, l'étape b) de réception consistant à recevoir une image courante du tissu oculaire, ladite image courante ayant été acquise par le système d'imagerie postérieurement à l'émission de la pluralité de faisceaux L.A.S.E.R. en un point d'échantillonnage respectif de chaque zone élémentaire.

14. Procédé de détermination selon la revendication 13, dans lequel l'étape de détermination inclut les sous-étapes suivantes pour chaque zone élémentaire :
- si une unique bulle de gaz est détectée :
• assigner à la valeur minimale, un multiple k de l'énergie du faisceau L.A.S.E.R. associé au point d'échantillonnage au niveau duquel la bulle de gaz est détectée, k étant un nombre décimal compris entre 1 et 2,
- si plusieurs bulles de gaz sont détectées :
• sélectionner parmi les points d'échantillonnage au niveau de chacun desquels une bulle de gaz est détectée, le point d'échantillonnage associé au faisceau L.A.S.E.R. d'énergie la plus faible, et
• assigner à la valeur minimale, un multiple k de l'énergie du faisceau L.A.S.E.R. associé audit point d'échantillonnage sélectionné, k étant un nombre décimal compris entre 1 et 2.

15. Procédé de détermination selon l'une quelconque des revendications 9 à 14, ***lequel*** comprend en outre l'étape suivante :
e) enregistrement par l'unité de traitement de données (90), de la valeur minimale déterminée pour chaque zone élémentaire (62) dans une mémoire.

## Patentansprüche

1. Vorrichtung (70) zur Bestimmung eines Mindestwerts an LASER-Energie, die zum Bilden einer Gasblase unter Verwendung einer Schneidvorrichtung, die eine Femtosekunden-LASER-Quelle (10) umfasst, in mindestens einer Elementarzone (62) notwendig ist, die sich in einer Schnittebene eines Augengewebes (60) erstreckt, wobei die Bestimmungsvorrichtung (70) ein Bildgebungssystem (80) für die Erfassung von Bildern umfasst, wobei die Bestimmungsvorrichtung (70) ferner eine Datenverarbeitungseinheit (90) umfasst, die Mittel für Folgendes umfasst:
a) Empfangen eines Bezugsbildes des Augengewebes, wobei das Bezugsbild durch das Bildgebungssystem (80) vor der Emission (210, 310) einer Vielzahl von LASER-Strahlen erfasst (200, 300) wurde, die dazu geeignet sind, auf mindestens einen Abtastpunkt von jeder Elementarzone (62) zu fokussieren, wobei jeder LASER-Strahl eine jeweilige Energie aufweist, die sich von denjenigen der anderen LASER-Strahlen der Vielzahl unterscheidet,
b) Empfangen mindestens eines aktuellen Bildes des Augengewebes (60), wobei jedes aktuelle Bild durch das Bildgebungssystem (80) nach der Emission (210, 310) von mindestens einem LASER-Strahl von der Vielzahl von LASER-Strahlen erfasst (220, 320) wurde,
und dann, für jede Elementarzone:
c) Ermitteln mindestens einer Gasblase, die in der Elementarzone gebildet ist, durch Vergleichen des Bezugsbildes mit jedem aktuellen Bild, wobei jede Gasblase einem jeweiligen LASER-Strahl zugehörig ist,
d) Bestimmen eines Mindestwerts an Energie, die zum Bilden einer Gasblase in der Elementarzone notwendig ist, in Abhängigkeit von der mindestens einen Gasblase.

2. Bestimmungsvorrichtung nach Anspruch 1, wobei das Bezugs- und das/die aktuelle/n Bild/er OCT-Bilder sind und die Mittel zum Ermitteln, für jedes aktuelle Bild, für Folgendes geeignet sind:
• Berechnen, an jedem Abtastpunkt, einer Schwankung der Stärke von rückgestreutem Licht, das von dem Bildgebungssystem (80) empfangen wird, zwischen den Pixeln des aktuellen Bildes und den Pixeln des Bezugsbildes,
• Vergleichen dieser berechneten Stärkenschwankung mit einem Schwellenwert, um die Bildung einer Gasblase zu identifizieren, wenn die berechnete Stärkenschwankung höher als der Schwellenwert ist.

3. Bestimmungsvorrichtung nach einem der Ansprüche 1 oder 2, wobei für jede Elementarzone jeder LASER-Strahl dazu geeignet ist, auf einen einzigen Abtastpunkt der Elementarzone zu fokussieren, wobei die Mittel zum Empfangen dazu geeignet sind, eine Vielzahl von aktuellen Bildern des Augengewebes zu empfangen, wobei jedes aktuelle Bild durch das Bildgebungssystem nach der Emission eines jeweiligen LASER-Strahls in jeder Elementarzone erfasst wurde.

4. Bestimmungsvorrichtung nach Anspruch 3, wobei die Mittel zum Bestimmen für jede Elementarzone für Folgendes geeignet sind:
- wenn eine Gasblase ermittelt wird:
• Zuweisen eines Vielfachen k der Energie des LASER-Strahls, der die Bildung der ermittelten Gasblase hervorgerufen hat, zu dem Mindestwert, wobei k eine Dezimalzahl zwischen 1 und 2 ist,
- wenn mehrere Gasblasen ermittelt werden:
• Auswählen des aktuellen Bildes, das dem LASER-Strahl mit der niedrigsten Energie zugehörig ist, unter den aktuellen Bildern, in denen eine Gasblase ermittelt wird, und
• Zuweisen eines Vielfachen k der Energie des LASER-Strahls, der dem ausgewählten aktuellen Bild zugehörig ist, zu dem Mindestwert, wobei k eine Dezimalzahl zwischen 1 und 2 ist.

5. Bestimmungsvorrichtung nach einem der Ansprüche 1 oder 2, wobei für jede Elementarzone jeder LASER-Strahl dazu geeignet ist, auf einen jeweiligen Abtastpunkt der Elementarzone zu fokussieren, wobei die Mittel zum Empfangen dazu geeignet sind, ein aktuelles Bild des Augengewebes zu empfangen, wobei das aktuelle Bild durch das Bildgebungssystem nach der Emission der Vielzahl von LASER-Strahlen an einem jeweiligen Abtastpunkt von jeder Elementarzone erfasst wurde.

6. Bestimmungsvorrichtung nach Anspruch 5, wobei die Mittel zum Bestimmen, für jede Elementarzone, geeignet sind zum:
- wenn eine einzige Gasblase ermittelt wird:
• Zuweisen eines Vielfachen k der Energie des LASER-Strahls, der dem Abtastpunkt zugehörig ist, in dessen Bereich die Gasblase ermittelt wird, zu dem Mindestwert, wobei k eine Dezimalzahl zwischen 1 und 2 ist,
- wenn mehrere Gasblasen ermittelt werden:
• Auswählen des Abtastpunkts, der dem LASER-Strahl mit der niedrigsten Energie zugehörig ist, unter jedem der Abtastpunkte, in deren Bereich eine Gasblase ermittelt wird, und
• Zuweisen eines Vielfachen k der Energie des LASER-Strahls, der dem ausgewählten Abtastpunkt zugehörig ist, zu dem Mindestwert, wobei k eine Dezimalzahl zwischen 1 und 2 ist.

7. Bestimmungsvorrichtung nach einem der Ansprüche 1 bis 6, wobei die Verarbeitungseinheit ferner Mittel umfasst zum:
e) Aufzeichnen des für jede Elementarzone (62) bestimmten Mindestwerts in einem Speicher.

8. Vorrichtung nach Anspruch 7, wobei das Augengewebe eine Menge von Elementarzonen umfasst, die sich in einer Vielzahl von Schnittebenen erstrecken, die jeweils mindestens eine Elementarzone (62) enthalten, wobei die Datenverarbeitungseinheit ferner dazu angepasst ist, die Schritte a) bis e) für jede Schnittebene zu wiederholen, um eine dreidimensionale Karte der Mindestwerte von zur Bildung von Gasblasen in der Menge der Elementarzonen (62) des Augengewebes (60) notwendiger Energie zu bestimmen.

9. Verfahren zur Bestimmung eines Mindestwerts an LASER-Energie, die zum Bilden einer Gasblase unter Verwendung einer Schneidvorrichtung, die eine Femtosekunden-LASER-Quelle (10) umfasst, in mindestens einer Elementarzone (62) notwendig ist, die sich in einer Schnittebene eines Augengewebes (60) erstreckt, wobei das Verfahren die folgenden Schritte umfasst:
a) Empfangen, durch eine Datenverarbeitungseinheit (90), eines Bezugsbildes des Augengewebes (60), wobei das Bezugsbild durch ein Bildgebungssystem (80) vor der Emission (210, 310) einer Vielzahl von LASER-Strahlen erfasst (200, 300) wurde, die dazu geeignet sind, auf mindestens einen Abtastpunkt von jeder Elementarzone (62) zu fokussieren, wobei jeder LASER-Strahl eine jeweilige Energie aufweist, die sich von denjenigen der anderen LASER-Strahlen der Vielzahl unterscheidet,
b) Empfangen, durch die Datenverarbeitungseinheit (90), mindestens eines aktuellen Bildes des Augengewebes (60), wobei jedes aktuelle Bild durch das Bildgebungssystem (80) nach der Emission (210, 310) von mindestens einem LASER-Strahl von der Vielzahl von LASER-Strahlen erfasst (220, 320) wurde,
und dann, für jede Elementarzone, Durchführen der folgenden Schritte durch die Datenverarbeitungseinheit (90):
c) Ermitteln (230, 330) mindestens einer Gasblase, die in der Elementarzone gebildet ist, durch Vergleichen des Bezugsbildes mit jedem aktuellen Bild, wobei jede Gasblase einem jeweiligen LASER-Strahl zugehörig ist,
d) Bestimmen (240, 250; 340, 350) eines Mindestwerts an Energie, die zum Bilden einer Gasblase in der Elementarzone notwendig ist, in Abhängigkeit von der mindestens einen ermittelten Gasblase.

10. Bestimmungsverfahren nach Anspruch 9, wobei das Bezugs- und das/die aktuelle/n Bild/er OCT-Bilder sind, wobei der Schritt des Ermittelns (230, 330), für jedes aktuelle Bild, die Teilschritte umfasst, die bestehen im:
• Berechnen, an jedem Abtastpunkt, einer Schwankung der Stärke von rückgestreutem Licht, das von dem Bildgebungssystem (80) empfangen wird, zwischen den Pixeln des aktuellen Bildes und den Pixeln des Bezugsbildes,
• Vergleichen dieser berechneten Stärkenschwankung mit einem Schwellenwert, um die Bildung einer Gasblase zu identifizieren, wenn die berechnete Stärkenschwankung höher als der Schwellenwert ist.

11. Bestimmungsverfahren nach einem der Ansprüche 9 bis 10, wobei für jede Elementarzone jeder LASER-Strahl dazu geeignet ist, auf einen einzigen Abtastpunkt der Elementarzone zu fokussieren, wobei der Schritt b) des Empfangens im Empfangen einer Vielzahl von aktuellen Bildern des Augengewebes besteht, wobei jedes aktuelle Bild durch das Bildgebungssystem nach der Emission eines jeweiligen LASER-Strahls in jeder Elementarzone erfasst wurde.

12. Bestimmungsverfahren nach Anspruch 11, wobei der Schritt des Bestimmens für jede Elementarzone die folgenden Unterschritte umfasst:
- wenn eine Gasblase ermittelt wird:
• Zuweisen eines Vielfachen k der Energie des LASER-Strahls, der die Bildung der ermittelten Gasblase hervorgerufen hat, zu dem Mindestwert, wobei k eine Dezimalzahl zwischen 1 und 2 ist,
- wenn mehrere Gasblasen ermittelt werden:
• Auswählen des aktuellen Bildes, das dem LASER-Strahl mit der niedrigsten Energie zugehörig ist, unter den aktuellen Bildern, in denen eine Gasblase ermittelt wird, und
• Zuweisen eines Vielfachen k der Energie des LASER-Strahls, der dem ausgewählten aktuellen Bild zugehörig ist, zu dem Mindestwert, wobei k eine Dezimalzahl zwischen 1 und 2 ist.

13. Bestimmungsverfahren nach einem der Ansprüche 9 oder 10, wobei für jede Elementarzone jeder LASER-Strahl dazu geeignet ist, auf einen jeweiligen Abtastpunkt der Elementarzone zu fokussieren, der Schritt b) des Empfangens darin besteht, ein aktuelles Bild des Augengewebes zu empfangen, wobei das aktuelle Bild durch das Bildgebungssystem nach der Emission der Vielzahl von LASER-Strahlen an einem jeweiligen Abtastpunkt von jeder Elementarzone erfasst wurde.

14. Bestimmungsverfahren nach Anspruch 13, wobei der Bestimmungsschritt für jede Elementarzone die folgenden Unterschritte umfasst:
- wenn eine einzige Gasblase ermittelt wird:
• Zuweisen eines Vielfachen k der Energie des LASER-Strahls, der dem Abtastpunkt zugehörig ist, in dessen Bereich die Gasblase ermittelt wird, zu dem Mindestwert, wobei k eine Dezimalzahl zwischen 1 und 2 ist,
- wenn mehrere Gasblasen ermittelt werden:
• Auswählen des Abtastpunkts, der dem LASER-Strahl mit der niedrigsten Energie zugehörig ist, unter jedem der Abtastpunkte, in deren Bereich eine Gasblase ermittelt wird, und
• Zuweisen eines Vielfachen k der Energie des LASER-Strahls, der dem ausgewählten Abtastpunkt zugehörig ist, zu dem Mindestwert, wobei k eine Dezimalzahl zwischen 1 und 2 ist.

15. Bestimmungsverfahren nach einem der Ansprüche 9 bis 14, das ferner den folgenden Schritt umfasst:
e) Speichern des für jede Elementarzone (62) bestimmten Mindestwerts durch die Datenverarbeitungseinheit (90) in einem Speicher.

## Claims

1. A determination device (70) for determining a minimum value of L.A.S.E.R. energy necessary to form, by using a cutting apparatus including a femtosecond L.A.S.E.R. source (10), a gas bubble in at least one elementary area (62) extending in a cutting plane of an ocular tissue (60), the determination device (70) including an imaging system (80) for the acquisition of images, the determination device (70) further comprising a data processing unit (90) including means for:
a) Receiving a reference image of the ocular tissue, said reference image having been acquired (200, 300) by the imaging system (80) prior to the emission (210, 310) of a plurality of L.A.S.E.R. beams able to focus at least at one sampling point of each elementary area (62), each L.A.S.E.R. beam having a respective energy different from the other L.A.S.E.R. beams of the plurality,
b) Receiving at least one current image of the ocular tissue (60), each current image having been acquired (220, 320) by the imaging system (80) subsequently to the emission (210, 310) of at least one L.A.S.E.R. beam of the plurality of L.A.S.E.R. Beams,
then for each elementary area:
c) Detecting by comparing the reference image with each current image, at least one gas bubble formed in the elementary area, each gas bubble being associated with a respective L.A.S.E.R. beam,
d) Determining, based on the at least one gas bubble, a minimum value of energy necessary for the formation of a gas bubble in the elementary area.

2. The determination device according to claim 1, ***wherein*** the reference and current images are OCT images, the detection means being, for each current image, able to:
• calculate in each sampling point, an intensity variation of backscattered light received by the imaging system (80) between the pixels of the current image and the pixels of the reference image,
• compare this calculated intensity variation with a threshold value to identify the formation of a gas bubble if the calculated intensity variation is greater than the threshold value.

3. The determination device according to any one of claims 1 or 2, ***wherein*** for each elementary area, each L.A.S.E.R. beam is able to focus at a single sampling point of the elementary area, the receiving means being able to receive a plurality of current images of the ocular tissue, each current image having been acquired by the imaging system subsequently to the emission of a respective L.A.S.E.R. beam in each elementary area.

4. The determination device according to claim 3, ***wherein*** the determination means are, for each elementary area, able to:
- if a single gas bubble is detected:
• assign to the minimum value a multiple k of the energy of the L.A.S.E.R. beam having induced the formation of the detected gas bubble, k being a decimal number comprised between 1 and 2,
- if several gas bubbles are detected:
• select among the current images in which a gas bubble is detected, the current image associated with the L.A.S.E.R. beam of lowest energy, and
• assign to the minimum value a multiple k of the energy of the L.A.S.E.R. beam associated with the selected current image, k being a decimal number comprised between 1 and 2.

5. The determination device according to any one of claims 1 or 2, ***wherein*** for each elementary area, each L.A.S.E.R. beam is able to focus at a respective sampling point of the elementary area, the receiving means being able to receive a current image of the ocular tissue, said current image having been acquired by the imaging system subsequently to the emission of the plurality of L.A.S.E.R. beams at a respective sampling point of each elementary area.

6. The determination device according to claim 5, ***wherein*** the determination means are, for each elementary area, able to:
- if a single gas bubble is detected:
• assign to the minimum value a multiple k of the energy of the L.A.S.E.R. beam associated with the sampling point at which the gas bubble is detected, k being a decimal number comprised between 1 and 2,
- if several gas bubbles are detected:
• select among the sampling points at each of which a gas bubble is detected, the sampling point associated with the L.A.S.E.R. beam of lowest energy, and
• assign to the minimum value a multiple k of the energy of the L.A.S.E.R. beam associated with said selected sampling point, k being a decimal number comprised between 1 and 2.

7. The determination device according to any one of claims 1 to 6, ***wherein*** the processing unit further comprises means for:
e) recording the minimum value determined for each elementary area (62) in a storage unit.

8. The determination device according to claim 7, ***wherein*** the ocular tissue comprises a set of elementary areas extending in a plurality of cutting planes each containing at least one elementary area (62), the data processing unit further being adapted to reiterate steps a) to e) for each cutting plane in order to determine a three-dimensional map of the minimum values of energy necessary for the formation of gas bubbles in the set of the elementary areas (62) of the ocular tissue (60).

9. A determination method for determining a minimum value of L.A.S.E.R. energy necessary to form, by using a cutting apparatus including a femtosecond L.A.S.E.R. source (10), a gas bubble in at least one elementary area (62) extending in a cutting plane of an ocular tissue (60), the method comprising the following steps:
a) Receiving by a data processing unit (90) of a reference image of the ocular tissue (60), said reference image having been acquired (200, 300) by an imaging system (80) prior to the emission (210, 310) of a plurality of L.A.S.E.R. beams able to focus at least at one sampling point of each elementary area (62), each L.A.S.E.R. beam having a respective energy different from the other L.A.S.E.R. beams of the plurality of L.A.S.E.R. beams,
b) Receiving by the data processing unit (90) of at least one current image of the ocular tissue (60), each current image having been acquired (220, 320) by the imaging system (80) subsequently to the emission (210, 310) of at least one L.A.S.E.R. beam of the plurality of L.A.S.E.R. beams,
then for each elementary area, implementing the following steps by the data processing unit (90):
c) Detecting (230, 330) by comparing the reference image with each current image, of at least one gas bubble formed in the elementary area, each gas bubble being associated with a respective L.A.S.E.R. beam,
d) Determining (240, 250; 340, 350) based on the at least one detected gas bubble, of a minimum value of energy necessary for the formation of a gas bubble in the elementary area.

10. The determination method according to claim 9, ***wherein*** the reference and current images are OCT images, the detection step (230, 330) comprising, for each current image, the sub-steps consisting in:
• calculating in each sampling point, an intensity variation of backscattered light received by the imaging system (80) between the pixels of the current image and the pixels of the reference image,
• comparing this calculated intensity variation with a threshold value to identify the formation of a gas bubble if the calculated intensity variation is greater than the threshold value.

11. The determination method according to any one of claims 9 or 10, ***wherein*** for each elementary area, each L.A.S.E.R. beam is able to focus at a single sampling point of the elementary area, the receiving step b) consisting in receiving a plurality of current images of the ocular tissue, each current image having been acquired by the imaging system subsequently to the emission of a respective L.A.S.E.R. beam in each elementary area.

12. The determination method according to claim 11, ***wherein*** the determination step includes the following sub-steps for each elementary area:
- if a single gas bubble is detected:
• assigning to the minimum value a multiple k of the energy of the L.A.S.E.R. beam having induced the formation of the detected gas bubble, k being a decimal number comprised between 1 and 2,
- if several gas bubbles are detected:
• selecting among the current images in which a gas bubble is detected, the current image associated with the L.A.S.E.R. beam of lowest energy, and
• assigning to the minimum value a multiple k of the energy of the L.A.S.E.R. beam associated with the selected current image, k being a decimal number comprised between 1 and 2.

13. The determination method according to any one of claims 9 or 10, ***wherein*** for each elementary area, each L.A.S.E.R. beam is able to focus at a respective sampling point of the elementary area, the receiving step b) consisting in receiving a current image of the ocular tissue, said current image having been acquired by the imaging system subsequently to the emission of the plurality of L.A.S.E.R. beams at a respective sampling point of each elementary area.

14. The determination method according to claim 13, ***wherein*** the determination step includes the following sub-steps for each elementary area:
- if a single gas bubble is detected:
• assigning to the minimum value a multiple k of the energy of the L.A.S.E.R. beam associated with the sampling point at which the gas bubble is detected, k being a decimal number comprised between 1 and 2,
- if several gas bubbles are detected:
• selecting among the sampling points at each of which a gas bubble is detected, the sampling point associated with the L.A.S.E.R. beam of lowest energy, and
• assigning to the minimum value a multiple k of the energy of the L.A.S.E.R. beam associated with said selected sampling point, k being a decimal number comprised between 1 and 2.

15. The determination method according to any one of claims 9 to 14, *which* further comprises the following step:
e) recording by the data processing unit (90) the minimum value determined for each elementary area (62) in a storage unit.
